# EUROPEAN PATENT SPECIFICATION

(11) **EP 2 832 325 B1**
(45) Date of publication and mention of the grant of the patent: **08.06.2016**
(21) Application number: 13767735.7
(22) Date of filing: 28.03.2013
(51) Int. Cl.: A61F 13/49, A61F 13/494

(54) **DISPOSABLE DIAPER**
WEGWERFWINDEL
COUCHE JETABLE

(30) Priority: 30.03.2012 JP 2012083056
(43) Date of publication of application: 04.02.2015
(73) Proprietor: Unicharm Corporation, Ehime 799-0111 (JP)
(72) Inventor: SAKAGUCHI, Satoru, Kanonji-shi Kagawa 769-1602 (JP)
(74) Representative: Dolleymores
(86) International application number: PCT/JP2013/059329
(87) International publication number: WO 2013/147054

(56) References cited:
- EP-A1- 1 228 741
- EP-A1- 1 902 694
- EP-A2- 1 384 460
- JP-A- H1 147 189
- JP-A- H10 243 961
- JP-A- 2006 042 941
- JP-A- 2006 166 941
- US-A1- 2010 191 212

## Description

### [Technical Field]

The present invention relates to a disposable diaper including side flaps at side edges in the product widthwise direction of an absorbent body, and, in particular, to a disposable diaper which attempts to reduce the stimulation on the skin in the vicinity of leg hole portions of a wearer, caused by elastic members provided to the side flaps.

### [Background Art]

Conventionally, various disposable diapers have been devised to realize a good fit while ensuring to prevent leakage of bodily waste. For example, there is known a disposable diaper configured so that an interval in a front waistline unit between a pair of leg hole elastic members is larger than an interval in a back waistline unit between these elastic members (see Patent Literature 1).

EP 1384460 discloses a diaper having leg hole stretch regions with a constant width.

According to the above disposable diaper, a pocket (room) is formed in the front waistline unit, so that an absorber having enough size to fully absorb bodily waste can be arranged in the front waistline unit. Therefore, it is contemplated that the leakage can be effectively prevented from the front waistline unit which practically receives a larger volume of urine excreted.

### [Citation List]

### [Patent Literature]

[PTL 1] Japanese Unexamined Patent Application Publication No. H11-47189 (Fig. 1)

### [Summary of Invention]

### [Technical Problem]

However, the conventional disposable diaper described above has the following problems. That is, since the leg hole elastic member is provided at each side end in the product widthwise direction, it is prone to bite into the skin of a wearer, thereby causing a problem of stimulation on the skin of a wearer. It becomes more of a problem especially in the case of a disposable diaper for infants and toddlers since the skin of infants and toddlers has poor resistance to this stimulation.

Therefore, the present invention has been achieved in view of the above-described situation, and an object thereof is to provide a disposable diaper which reduces the greater stimulation on the skin in the vicinity of the leg hole portions of a wearer, while enhancing contact in the vicinity of leg holes.

### [Solution to Problem]

An aspect of the present invention is summarized as a disposable diaper (disposable diaper 10), including: a front waistline unit (front waistline unit 20); a back waistline unit (back waistline unit 30); a crotch unit (crotch unit 25); a vertically long absorbent body (absorbent body 15) including a liquid-retaining absorber (absorber 40); a side flap (side flap 70) provided at each side edge in a product widthwise direction of the absorbent body; a leg hole opening unit (leg hole opening unit 35) that is concaved towards a center in the product widthwise direction of the absorbent body, and is formed at the each side edge in the product widthwise direction of the absorbent body; wherein the disposable diaper includes a sheet-like leg hole stretch unit (leg hole stretch unit 100) provided at a side end, in the product widthwise direction (product widthwise direction W), of the side flap, extending in a product longitudinal direction (product longitudinal direction L), and being partially stretchable at least in the product longitudinal direction; a side edge, in the product widthwise direction, of the leg hole stretch unit is in an undulating shape in the product longitudinal direction; a distance (for example, distance d1) between the side edge, in the product widthwise direction, of the leg hole stretch unit and a straight line (center line CL), passing through a center in the product widthwise direction of the disposable diaper and extending in parallel to the product longitudinal direction, changes from the front waistline unit to the back waistline unit; a plurality of concave units (concave unit 140, concave unit 160), where an amount of change along the distance starts increasing instead of decreasing , are provided closer to the back waistline unit than a center, in the product longitudinal direction, of the disposable diaper; and a width, in the product widthwise direction, of the leg hole stretch unit provided with the concave units is smaller than a width, in the product widthwise direction, of the leg hole stretch unit other than the concave units.

### [Advantageous Effects of Invention]

According to the features of the present invention, it is possible to provide a disposable diaper which reduces the greater stimulation on the skin in the vicinity of the leg hole portions of a wearer, while enhancing contact in the vicinity of leg holes.

### [Brief Description of Drawings]

[Fig. 1] Fig. 1 is an exploded plan view of a disposable diaper 10 according to an embodiment of the present invention.
[Fig. 2] Fig. 2 is an enlarged plan view of a leg hole stretch unit 100 according to the embodiment of the present invention.
[Fig. 3] Fig. 3 is a diagram illustrating schematically a cross section of the disposable diaper 10 taken along the line F3-F3 shown in Fig. 1.
[Fig. 4] Fig. 4 is a diagram illustrating schematically a state in which the disposable diaper 10 according to the embodiment of the present invention is worn by a wearer.
[Fig. 5] Fig. 5 is an enlarged plan view of a leg hole stretch unit 100A according to a modification of the present invention.

### [Description of Embodiments]

Hereinafter, an embodiment of a disposable diaper according to the present invention is described with reference to accompanying drawings. In the following description of the drawings, the same or similar reference numerals are used to designate the same or similar units. It will be appreciated that the drawings are schematically shown and the ratio and the like of each dimension are different from the real ones.

Accordingly, specific dimensions should be determined in consideration of the explanation below. Moreover, among the drawings, the respective dimensional relations or ratios may differ.

### (1) Overall Schematic Configuration of Disposable Diaper

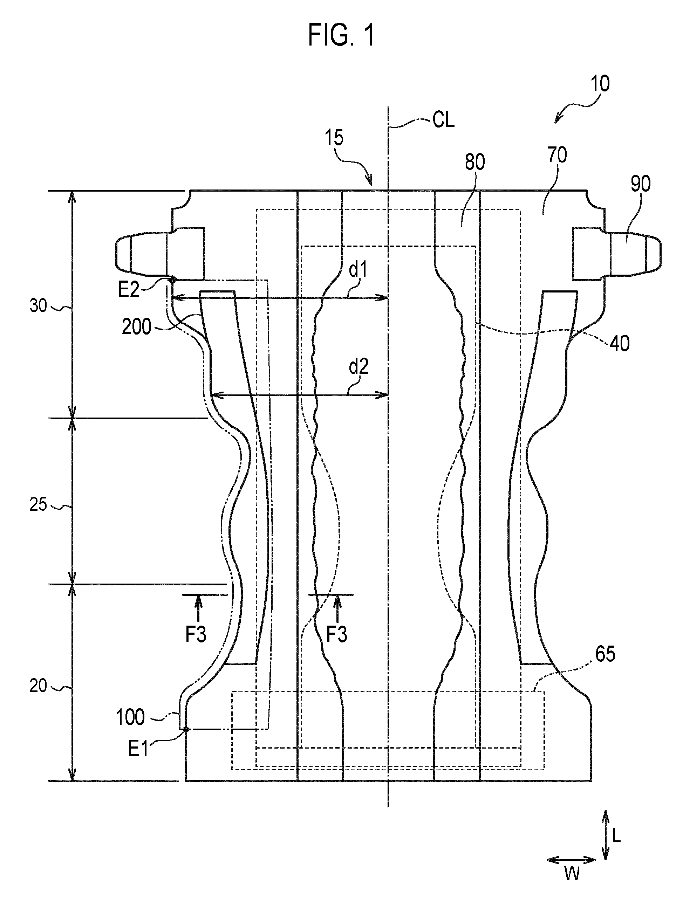

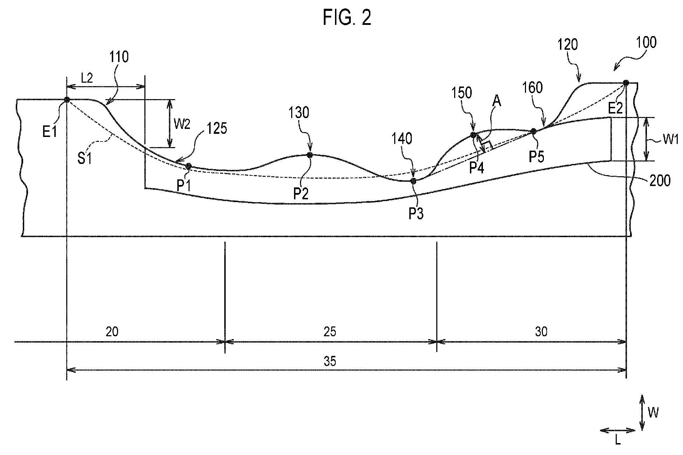

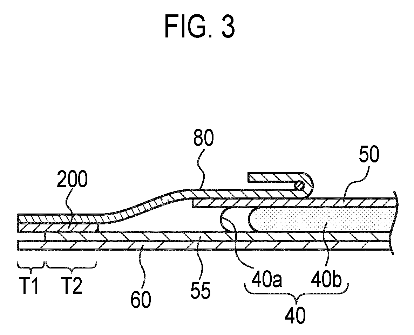

Fig. 1 is an exploded plan view of a disposable diaper 10 according to the present embodiment. Fig. 2 is an enlarged plan view of a leg hole stretch unit 100. Fig. 3 is a diagram illustrating schematically a cross section of the disposable diaper 10 taken along the line F3-F3 shown in Fig. 1.

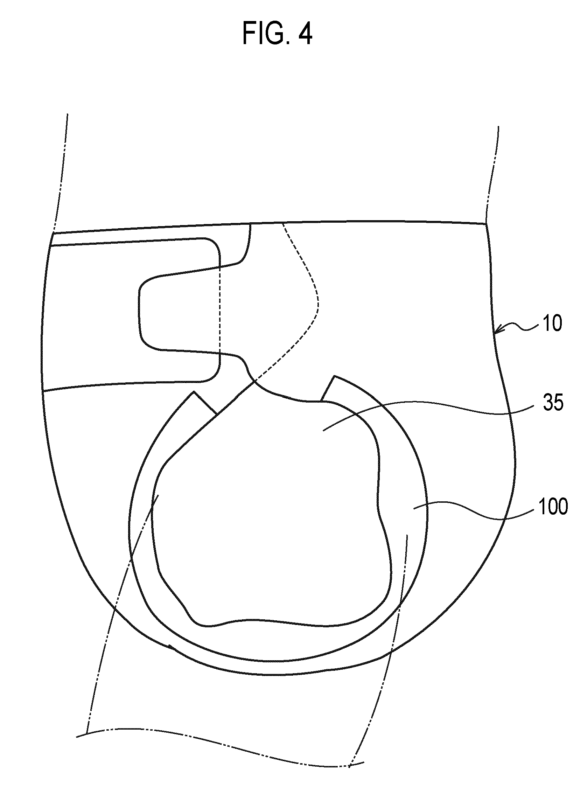

As shown in Figs. 1 to 3, the disposable diaper 10 has a front waistline unit 20, a crotch unit 25, and a back waistline unit 30. The front waistline unit 20 is a part coming in contact with the front waistline portion of a wearer. Furthermore, the back waistline unit 30 is a part coming in contact with the back waistline portion of the wearer. The crotch unit 25 is positioned between the front waistline unit 20 and the back waistline unit 30. An absorbent body 15 is configured by the front waistline unit 20, the crotch unit 25, and the back waistline unit 30. Furthermore, a pair of leg hole concave units 35 (see Fig. 4) are formed in the disposable diaper 10. Specifically, the leg hole concave units 35 are formed at side edges in a product widthwise direction W of the absorbent body 15 and are concaved towards the center in the product widthwise direction W. The leg hole concave units 35 form openings formed from the front waistline unit 20 to the back waistline unit 30 by engaging fastening tapes 90 provided at the back waistline unit 30 with the front waistline unit 20, i.e., with a target tape 65, at the time of wearing the disposable diaper 10 by a wearer.

The leg hole concave units 35 are openings which are formed from the front waistline unit 20 to the back waistline unit 30 by engaging the fastening tapes 90 provided at the back waistline unit 30 with the front waistline unit 20 (target tape) at the time of wearing the disposable diaper 10 by a wearer.

In the present embodiment, the direction from the front waistline unit 20 towards the back waistline unit 30 is called the product longitudinal direction L, and the direction perpendicular to the product longitudinal direction L is called the product widthwise direction W.

The disposable diaper 10 includes an absorber 40 spanning the crotch unit 25 and extending in the front waistline unit 20 and the back waistline unit 30. The absorbent body 15 has a vertically long shape including a liquid-retaining absorber 40. The absorber 40 is made up of an absorbent core 40a and a core wrap 40b.

The absorber 40 is the same as in a conventional disposable diaper, and can be configured appropriately by using popular components and materials, such as ground pulp and high absorbent polymer. The absorbent core 40a is wrapped by the sheet-like core wrap 40b. The core wrap 40b is a sheet for wrapping the absorbent core 40a. A part of at least the skin surface contact side of the core wrap 40b is configured by various fibrous nonwoven fabrics or a tissue sheet having liquid-permeability. For example, an air-through fibrous nonwoven fabric, a spunbond nonwoven fabric, or an SMS (spunbond - meltblown - spunbond) nonwoven cloth having a mass of approximately 10 to 30 g/m², or a tissue sheet having a mass of approximately 10 to 30 g/m² can be used.

A liquid-permeable topsheet 50 is provided at the top side (skin contact surface side) of the absorber 40. Furthermore, a liquid-impermeable leakage-preventing film 55 is provided at the back side (non-skin contact surface side) of the absorber 40. A backsheet 60 made of a nonwoven fabric is provided at the back side of the leakage-preventing film 55.

A side flap 70 is provided at each side edge in the product widthwise direction W of the absorbent body 15, from the front waistline unit 20 to the back waistline unit 30. The side flap 70 is made of one or two or more pieces of nonwoven fabric overlapping one another. Furthermore, a pair of side flaps 70 are provided with the fastening tapes 90, respectively. Specifically, the side flaps 70 formed in the back waistline unit 30 are provided with the pair of fastening tapes 90 which are engageable with the front waistline unit 20.

A pair of left-right leg hole stretch units 100 are formed at side ends in the product widthwise direction W of the side flaps 70. The leg hole stretch units 100 extend in the product longitudinal direction L and are partially stretchable at least in the product longitudinal direction L. In the present embodiment, the leg hole stretch units 100 are regions including stretch sheets 200 (see Fig. 1), and side edges in the product widthwise direction of the leg hole stretch units 100 are in an undulating shape in the product longitudinal direction L. The stretch sheets 200 are made of a stretchable nonwoven fabric sheet.

Furthermore, a pair of leg side gathers 80 extending in the product longitudinal direction L are provided at the inner side of the pair of leg hole stretch units 100 (towards the center in the product widthwise direction W). It should be noted that the disposable diaper 10 may be provided with waist gathers arranged in the front waistline unit 20 and the back waistline unit 30 in the product widthwise direction W.

### (2) Shape of Leg Hole Stretch Unit 100

Next, the shape of the leg hole stretch units 100 and the like are explained in details. As shown in Fig. 2, the leg hole stretch units 100 are formed in end edges of the leg hole concave units 35 of the disposable diaper 10, respectively. As described above, the side edges in the product widthwise direction of the leg hole stretch units 100 undulate in the product longitudinal direction L. Specifically, because of formation of inflection points P1 to P5 in the leg hole stretch unit 100, the side edges in the product widthwise direction W of the leg hole stretch units 100 become a undulating shape. Therefore, each of the side ends in the product widthwise direction of the leg hole stretch units 100 is convexo-concave in the product widthwise direction W with respect to a curved line S1 along the leg holes of a wearer of the disposable diaper 10.

More specifically, a distance (distances d1, d2) between each of side edges in the product widthwise direction W of the leg hole stretch units 100 and a center line CL (see Fig. 1), as a straight line passing through the center in the product widthwise direction W and extending in parallel to the product longitudinal direction, changes from the front waistline unit 20 to the back waistline unit 30. The plural number of inflection points (inflection points P2, P4) at which the amount of change along this distance starts decreasing instead of increasing and the plural number of inflection points (inflection points P1, P3, P5) at which the amount of change along this distance starts increasing instead of decreasing, are respectively provided.

Here, the distances are measured, when the disposable diaper 10 is in an open expanded condition.

A method of manufacturing the disposable diaper 10 includes at least a component-forming step, a component-loading step, a leg-hole-forming step, and a cutting step. In the component-forming step, components making up the disposable diaper 10 are formed. Specifically, for example, absorbent materials are laminated to form the absorber 40, and after arranging a continuous band-shaped elastic member in a predetermined shape, the elastic member is cut into an individual product length to form a stretch sheet 200.

In the component-loading step, on a web making up an exterior sheet, the components making up the disposable diaper are loaded, including, e.g., the stretch sheet 200, other webs such as a web configuring a topsheet 50, a water-resistive sheet, and the absorber.

In the leg-hole-forming step, the topsheet 50 and the exterior sheet are cut along the concave units making up the leg hole regions outside the stretch sheet 200 in the product widthwise direction W. In this manner, the leg hole regions arranged along the legs of a wearer are formed.

In the cutting step, a continuous body in which the topsheet 50, the exterior sheet, the absorber 40, and the stretch sheet 200 are arranged is cut into a size of a single product in the product widthwise direction W. In this manner, the disposable diaper 10 is manufactured.

Furthermore, in the present embodiment, a width W1 along the product widthwise direction W of the leg hole stretch units 100 in the natural state is preferably 5 mm to 45 mm, more preferably 12.5 m to 40mm. It should be noted that the natural state means a case where the disposable diaper 10 is taken out of a package and kept in ambient atmosphere having a temperature of 20°C ± 2°C, and a relative humidity of 60% ± 5% RH for 60 minutes, and then is measured. Furthermore, the ratio of expansion and contraction of the leg hole stretch units 100 is set to 1.5 times to 2.4 times.

Here, the widths are measured, when the disposable diaper 10 is in an open expanded condition.

The ratio of expansion and contraction implies the extent of expansion and contraction of the leg hole stretch units 100 in the product widthwise direction W, and is stipulated as below:

Ratio of expansion and contraction = (Length of the leg hole stretch unit 100 of the product in the maximum extended state) / (Length of the leg hole stretch unit 100 of the product in the natural state)

The length in the maximum extended state is obtained by measuring a length in the product widthwise direction W of the leg hole stretch units 100 as a measuring object at the time when the product is extended to the maximum extent so that the product is not destroyed, in the product (disposable diaper 10) provided with the leg hole stretch units 100 as a measuring object. Furthermore, the length in the natural state as a contracted state is obtained by measuring a length along the product widthwise direction W of the leg hole stretch units 100 in a state in which the product is placed on a flat surface while minimizing the convexities and concavities resulting from gathers, with as little load as possible, the load being applied to cause extension in the product widthwise direction. In a case like the present embodiment, in which the leg hole stretch units 100 are curved, it is preferable that a length be measured in each of the above-described states along a line of an inner end in the product width direction W of the stretch sheet 200.

Amplitude A in the product widthwise direction W at the undulating parts formed in the leg hole stretch units 100 is preferably 2.5 mm to 20 mm. In a case where a predetermined inflection point (for example, the inflection point P3) is used as a reference, the amplitude A is a distance between this reference inflection point and a position which is perpendicular to a straight line passing through two inflection points (for example, the inflection points P4, P5) adjacent to this reference inflection point and passes through this reference inflection point. It should be noted that a start point of an amplitude of a convex unit 110 is a position (E1 shown in Figs. 1 and 2) of the intersection point between the center, in the product longitudinal direction L, of the target tape 65 and each of the side edges of the side flaps 70 while a start point of an amplitude of a convex unit 120 is a position (E2 shown in Figs. 1 and 2) of a proximal end, at the crotch unit 25 side, of the fastening tape 90.

Furthermore, the amplitude A is a distance between the aforementioned inflection point and position which are measured in the disposable diaper 10 in an expanded and extended state. In a case of the amplitude A being less than 2.5 mm, convexo-concave regions cannot be formed enough in the leg hole stretch units 100, thereby making it difficult to reduce the pressure on the skin at the time of wearing the disposable diaper 10. Furthermore, the amplitude A being greater than 20 mm tends to invite a situation in which the leg hole stretch unit 100 is sandwiched between the body of a wearer and the disposable diaper 10 in association with a movement of the wearer, so that each convexo-concave region cannot exhibit the effect of its function. Yet further, the convexities and concavities stand out too much, which makes a poor visual impression of the leg holes at the time of wearing the disposable diaper 10.

The expanded and extended state implies a state in which the entire disposable diaper 10 is expanded as shown in Fig. 1. Specifically, the disposable diaper 10 can be put in such a state by extending a region of the gathers designed to be expanded and contracted with the use of elastic elements, to an extent that the gathers disappear visually, and retaining the disposable diaper 10 on a flat board.

Furthermore, the amplitude A can be also confirmed by taking only a non-stretchable member (in the present embodiment, the nonwoven fabric forming the side top or the backsheet) forming the leg hole units, out of the disposable diaper 10 main body.

The front and back ends of each leg hole concave unit 35 are provided with convex units 110, 120 which are convexed towards the outer side in the product widthwise direction W as compared to the other parts of the leg hole concave unit 35.

Herein, Fig. 4 is a diagram illustrating schematically a state in which the disposable diaper 10 is worn by a wearer. The front and back ends of the leg hole concave unit 35 are, because stress is likely to concentrate therein, preferably conditioned to achieve more distribution of the pressure on the skin by formation of the convex units 110, 120. Specifically, since these are positioned close to the proximal ends of the fastening tapes 90 and the target tape 65 provided to the front waistline unit 20, while functioning as the front and back ends of the leg hole concave unit 35, the stress is likely to concentrate therein, and these are also regions which are overlapped one after the other at the time of wearing the disposable diaper 10, so that these ends are preferably convexed in order to distribute the pressure on the skin.

Further, a width W2 in the product widthwise direction W and a length L2 in the product longitudinal direction L of the convex unit 110 positioned closer to the front end of the leg hole concave unit 35 (or the convex unit 120 positioned closer to the back end of the leg hole concave unit 35) are preferably greater than a width in the product widthwise direction W and a length in the product longitudinal direction L of the convex unit 130 formed in the crotch unit 25. The convex unit 130 and the concave unit 140 formed in the crotch unit 25 are required to be placed along the body of a wearer in addition to reducing the pressure on the skin. On the other hand, positions closer to the front end and the back end of the leg hole concave unit 35 are required to reduce a greater pressure on the skin. Accordingly, a shape like in the present embodiment can reduce the load itself on the leg hole stretch units 100 caused by a movement of a wearer while preventing the pressure on the skin from concentrating in a specific part.

Each of concave units 125 is concaved towards the center side in the product widthwise direction W so as to follow the curved line S1 along the leg hole of a wearer, thereby easily following the inguinal portion of the wearer. Accordingly, the stretch sheets 200 have a better fit to a wearer, thereby allowing the front waistline unit 20 to the crotch unit 25 of the disposable diaper 10 to stably follow the body regardless of movement of the wearer. For example, even in a case where the legs of a wearer move so as to narrow a width of the absorber 40, the concave unit 125 continues to fit into the inguinal unit, thereby enabling the absorber 40 to have a large width without narrowing it. Accordingly, it is preferable to have a concave unit like the concave unit 125 in a position closer to the front waistline unit 20 than the center, in the product longitudinal direction L, of the disposable diaper 10.

Each of the convex units 130 is formed in a position corresponding to the crotch unit 25 of a wearer. The crotch unit 25 is sandwiched between the leg holes of a wearer and is a region having a narrow distance between the right and left leg holes, so that it is a part in which the leg hole stretch units 100 are prone to be wedged in the disposable diaper 10 by being sandwiched between the absorbent body 15 and the body of a wearer. Therefore, it is preferable that the body be covered by the leg hole stretch units 100 as widely as possible, and the convex unit 130 can further ensure prevention of leakage of bodily waste from the absorbent body 15. Furthermore, as described above, fitting of the concave unit 125 into the inguinal unit further prevents wedging of the convex unit 130.

The concave units 140, 160 are formed closer to the back waistline unit 30 than the center, in the product longitudinal direction L, of the disposable diaper 10. That is, the plurality of these concave units are formed closer to the back waistline unit 30 than the center, in the product longitudinal direction L, of the disposable diaper 10. The concave unit 140 is formed adjacent to the center, in the product longitudinal direction L, of the disposable diaper 10. The concave unit 160 is formed in the back waistline unit 30. Furthermore, in the leg hole stretch units 100, a part provided with the concave units 140, 160 has a smaller width in the product widthwise direction W than that of a part other than the concave units 140, 160. Specifically, in the leg hole stretch units 100, the part provided with the concave units 140, 160 has a smaller width in the product widthwise direction W than a width in the product widthwise direction W of the convex units 130, 150 or a width in a stretchable region (a width of a region designated as W1 in Fig. 2) at the outer end in the product widthwise direction W of the leg hole stretch units 100.

Specifically, the concave units 140, 160 are formed so that the amount of change along a distance from the center line CL starts increasing instead of decreasing, more specifically, the concave units 140, 160 are formed so as to include the inflection points P3, P5 at which the amount of change along a distance from the center line CL starts increasing instead of decreasing.

Each of convex units 150 convexed towards the outer side in the product widthwise direction W is formed between the concave units 140, 160. That is, the convex units 140, 160 are formed at a predetermined interval in the product longitudinal direction L. Such a shape can prevent a situation that the leg hole stretch units 100 (or the leg side gathers 80) get stuck in the body of a wearer, so the crotch unit cannot approach the crotch of a wearer and the disposable diaper is worn without covering the front waistline (abdominal portion), at the time of wearing the disposable diaper 10 on the body of a wearer with the use of the fastening tapes 90.

The concave unit 160 is formed in order to prevent the situation that the leg hole stretch units 100 at the back waistline unit 30 side get stuck in the body of a wearer and cannot be fixed in a proper position at the time when the fastening tapes 90, located at respective sides of the back waistline unit 30, are fixed to the target tape 65 and the situation that the disposable diaper 10 is worn with the leg hole stretch units 100 at the back waistline unit 30 being projected from the fastening tapes 90. If the leg hole stretch units 100 get stuck in the body of a wearer and the disposable diaper 10 is worn with the leg hole stretch units 100 being projected, a force is exerted in a direction of shifting the disposable diaper 10 downward in association with movement of the legs of a wearer, and the shifting is prone to occur even if the disposable diaper 10 is retained at the waistline with the use of the fastening tapes 90, so that this is not preferable.

The convex unit 150 is formed, as well as the adjacent concave units 140, 160, in order to reduce the pressure on the skin mainly in a position corresponding to the hip portion, to a position corresponding to the back of wearer. Furthermore, the convex unit 150 covers the hip portion of a wearer widely to result in improvement of the appearance and at the same time, by covering the body widely, the convex unit 150 gives a sense of security that the leakage of bodily fluid or positional shifting of the disposable diaper 10 hardly occurs.

Furthermore, in the present embodiment, as shown in Fig. 3, in a position corresponding to the front waistline unit 20 and/or the crotch unit 25, a thickness at the outer side in the product widthwise direction W of the leg hole stretch units 100 is preferably smaller than a thickness at the inner side in the product widthwise direction W of the leg hole stretch units 100. Specifically, a thickness of each side edge in the product widthwise direction W of each of the leg hole stretch units 100 is made smaller than the inner side in the product widthwise direction W by decreasing the number of laminated sheets in a position closer to the each side edge. In the present embodiment, as shown in Fig. 3, three sheets (the leg side gather 80, the stretch sheet 200, and the back sheet 60)are provided in the side edge part (T1 in Fig. 3) in the product widthwise direction W of the leg hole stretch unit 100. On the other hand, four sheets (with the addition of the leakage-preventing film 55) are provided in the inner part (T2 in Fig. 3) in the product widthwise direction W of the leg hole stretch unit 100.

As described above, by forming each of the side edge parts in the product widthwise direction W of the leg hole stretch units 100 thinner than the inner side in the product widthwise direction W, the leg hole stretch units 100 are easily brought in close contact, from the inner side towards the outer sides in the product widthwise direction W, at the time of wearing the disposable diaper 10. In a case where a thickness is made smaller as approaching each of the side edge parts in the product widthwise direction W of the leg hole stretch units 100, the leg hole stretch units 100 are easily brought in close contact with the skin, and especially by providing this thin part to a concave part of the body, such as the inguinal portion of a wearer, the fitting property or stability of the leg hole stretch units 100 can be improved in the inguinal portion. At the same time, the leg hole stretch units 100 can exhibit the effect of distributing the pressure to the skin. Accordingly, the fitting property or stability of the leg hole stretch units 100 can be improved in the inguinal portion, while a enabling the leg hole stretch units 100 to exhibit the effect of distributing the pressure to the skin.

Furthermore, the stretch sheet and non-stretchable sheet making up the leg hole stretch units 100 are joined together by means of pattern coating such as spiral coating or omega coating in which a linear adhesive is placed to have both a part having an adhesive and a part having no adhesive. Furthermore, the stretch sheet is a nonwoven fabric sheet made from stretchable fabrics having elastomer property while the non-stretchable sheet is also a nonwoven fabric sheet.

That is, no film sheets exist in the ends of the leg hole concave units 35. With these configurations, the ends of the leg hole concave units 35 allow the nonwoven fabric sheets to entwine the fabrics with each other, thereby staying unopened at the time of usage of the disposable diaper 10 and, at the same time, the entire ends of the leg hole concave units 35 are free from an adhesive, thereby being able to make the leg hole stretch units 100 softer.

Furthermore, by means of the coating described above, it is also possible to prevent stretchability of the stretchable sheet from being damaged by the non-stretchable sheet, thereby being able to enhance contact with the body of a wearer in the vicinity of the leg hole concave units 35.

### (3) Operation and Effect

As described above, each of the side edges in the product widthwise direction W of the leg hole stretch units 100 is in a undulating shape in the product longitudinal direction L, and a distance between each of the side edges in the product widthwise direction W of the leg hole stretch units 100 and the center line CL changes from the front waistline unit 20 to the back waistline unit 30, and the plural number of inflection points at which the amount of change along this distance starts decreasing instead of increasing, and the plural number of inflection at which the amount of change along this distance starts increasing instead of decreasing, are respectively provided.

This prevents a situation where the leg hole stretch units 100 (or the leg side gathers 80) get stuck in the body of wearer and the disposable diaper 10 is worn without covering the front waistline (abdominal portion), at the time of wearing the disposable diaper 10 on the body of a wearer with the use of the fastening tapes 90. As described above, the leg hole stretch units 100 (or the leg side gathers 80) hardly get stuck in the body of a wearer, so that the disposable diaper 10 can be worn while a position in which the fastening tapes 90 are engaged with the target tape 65 is placed even closer to the center in the product widthwise direction W, thereby being able to enhance contact with the waistline portion or the hip portion of a wearer. Furthermore, the leg hole stretch units 100 in a undulating shape distribute the pressure effectively even when they are brought in close contact with the skin of a wearer, so that the leg hole stretch units 100 can be prevented effectively from biting into the skin of a wearer, thereby being able to reduce the stimulation on the skin of a wearer. That is, according to the disposable diaper 10, the stimulation on the skin in the vicinity of the leg hole units of a wearer can be further reduced while enhancing adhesion around the leg holes.

In a comparative test between the disposable diaper 10 and the conventional disposable diaper without having the leg hole stretch units 100 in a undulating shape, it has been confirmed that a wearing mark (skin redness) is prevented from occurring in, for example, the inguinal portion of a wearer while improving the fitting property of the leg holes.

Furthermore, in the leg hole stretch units 100, the part provided with the concave units 140, 160 has a smaller width in the product widthwise direction W than that of the part other than the concave units 140, 160, so that, in the leg hole stretch units 100, the part provided with the concave units 140, 160 is stretched out more than the part other than the concave units 140, 160. In the leg hole stretch units 100, the part stretched out more than the part other than the concave units 140, 160 is contracted more greatly, so that the concave units 140, 160 can be brought in even closer contact with the leg holes of a wearer.

### (4) Other embodiments

As described above, the present invention is disclosed through the above embodiments of the present invention. However, it should not be interpreted that the statements and drawings constituting a part of the present disclosure limit the present invention. From this disclosure, a variety of alternate embodiments, examples, and applicable techniques will become apparent to one skilled in the art.

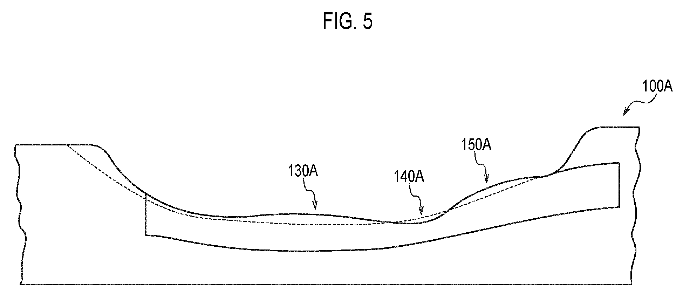

For example, a shape of the leg hole stretch units 100 may be modified as follows. Fig. 5 is an enlarged plan view of a leg hole stretch unit 100A according to a modification of the present invention. As shown in Fig. 5, convex units 130A, 150A and a concave unit 140A may be smaller in shape than the convex units 130, 150 and the concave unit 140 of the leg hole stretch unit 100. That is, a value of the amplitude A of the leg hole stretch unit 100A may be smaller than that of the leg hole stretch unit 100.

In the above-described embodiment, as shown in Fig. 3, each of the stretch sheets 200 making up the leg hole stretch units 100 extends up to each outer end in the product widthwise direction W. However, the stretch sheet 200 may not necessarily extend up to the outer end in the product widthwise direction W in consideration of manufacturability and the like. That is, the outer end, in the product widthwise direction W, of the stretch sheet 200 may be positioned at the inner side in the product widthwise direction W with respect to the outer end, in the product widthwise direction W, of the backsheet 60, the leg side gather 80, or the like. In such a case, a distance between the outer end, in the product widthwise direction W, of the stretch sheet 200 and the outer end, in the product widthwise direction W, of the backsheet 60, the leg side gather 80, or the like is preferably less than 7 mm.

Furthermore, in the above-described embodiment, the open-type disposable diaper provided with the fastening tapes 90 has been described as an example. However, the present invention can also be applied to a pant-type disposable diaper.

As described above, needless to say, the present invention includes various embodiments and the like not described here. Therefore, the technical range of the present invention is to be defined only by the inventive specific matter according to the applicable claims from the above description.

### [Reference Signs List]

10...Disposable diaper
15...Absorbent body
20... Front waistline unit
25... Crotch unit
30...Back waistline unit
35...Leg hole concave unit
40...Absorber
40a...Absorbent core
40b... Core wrap
50...Topsheet
55...Leakage-preventing film
60... Backsheet
65...Target tape
70...Side flap
80...Leg side gather
90...Fastening tape
100, 100A...Leg hole stretch unit
110, 120, 130, 130A, 150, 150A...Convex unit
125, 140, 140A, 160...Concave unit
200... Stretch sheet

## Claims

1. A disposable diaper 10, comprising:
a front waistline unit 20;
a back waistline unit 30;
a crotch unit 25;
a vertically long absorbent body 15 including a liquid-retaining absorber 40;
a side flap 70 provided at each side edge in a product widthwise direction of the absorbent body;
a leg hole opening unit 35 that is concaved towards a center in the product widthwise direction of the absorbent body, and is formed at the each side edge in the product widthwise direction of the absorbent body; wherein
the disposable diaper includes a sheet-like leg hole stretch unit 100 provided at a side end, in the product widthwise direction, of the side flap, extending in a product longitudinal direction, and being partially stretchable at least in the product longitudinal direction;
a side edge, in the product widthwise direction, of the leg hole stretch unit is in an undulating shape in the product longitudinal direction;
a distance between the side edge, in the product widthwise direction, of the leg hole stretch unit and a straight line passing through a center, in the product widthwise direction, of the disposable diaper and extending in parallel to the product longitudinal direction, changes from the front waistline unit to the back waistline unit;
a plurality of concave units where an amount of change along the distance starts increasing instead of decreasing are provided closer to the back waistline unit than a center, in the product longitudinal direction, of the disposable diaper; and
a width, in the product widthwise direction, of the leg hole stretch unit provided with the concave units is smaller than a width, in the product widthwise direction, of the leg hole stretch unit other than the concave units.

2. The disposable diaper according to claim 1, wherein
the concave units are formed adjacent to the center, in the product longitudinal direction, of the disposable diaper and are formed in the back waistline unit.

3. The disposable diaper according to claim 2, wherein
a convex unit, that is convexed towards an outer side in the product widthwise direction, is formed between the concave units.

4. The disposable diaper according to any one of claims 1 to 3, wherein
the leg hole stretch unit is provided along an end edge of the leg hole opening of the disposable diaper; and
the side edge, in the product widthwise direction, of the leg hole stretch unit forms the undulating shape by formation of an inflection point in the leg hole stretch unit.

5. The disposable diaper according to any one of claims 1 to 4, wherein
a width in the product widthwise direction of the leg hole stretch unit in a natural state is set to 5 mm to 45 mm.

6. The disposable diaper according to any one of claims 1 to 5, wherein
a ratio of expansion and contraction of the leg hole stretch unit is set to 1.5 times to 2.4 times.

7. The disposable diaper according to any one of claims 1 to 6, wherein
an amplitude in the product widthwise direction, at a part in an undulating shape formed in the leg hole stretch unit, is set to 2.5 mm to 20 mm.

8. The disposable diaper according to any one of claims 1 to 7, wherein
the side flap, provided at the back waistline unit, includes a pair of fastening tapes capable of engaging with the front waistline unit.

## Patentansprüche

1. Wegwerfwindel 10, umfassend:
eine vordere Tailleneinheit 20;
eine rückwärtige Tailleneinheit 30:
eine Schritteinheit 25;
einen vertikal langen saugfähigen Körper 15 mit einem Flüssigkeit haltenden Absorber 40;
eine Seitenklappe 70 an jedem Seitenrand in einer Produktbreitenrichtung des saugfähigen Körpers;
eine Beinlocheinheit 35, die in Richtung einer Mitte in der Produktbreitenrichtung des saugfähigen Körpers konkav ist und an jedem Seitenrand in der Produktbreitenrichtung des saugfähigen Körpers ausgebildet ist; wobei
die Wegwerfwindel eine folienartige Beinloch-Stretcheinheit 100 umfasst, die an einem Seitenende der Seitenklappe in der Produktbreitenrichtung vorgesehen ist, sich in einer Produktlängsrichtung erstreckt und mindestens in der Produktlängsrichtung teilweise dehnbar ist;
ein Seitenrand der Beinloch-Stretcheinheit in der Produktbreitenrichtung eine wellige Form in der Produktlängsrichtung hat;
der Abstand zwischen dem Seitenrand der Beinloch-Stretcheinheit in der Produktbreitenrichtung und einer geraden Linie, die durch eine Mitte der Wegwerfwindel in der Produktbreitenrichtung geht und sich parallel zur Produktlängsrichtung erstreckt, sich von der vorderen Tailleneinheit zur rückwärtigen Tailleneinheit hin ändert;
näher bei der rückwärtigen Tailleneinheit als bei einer Mitte der Wegwerfwindel in der Produktlängsrichtung eine Vielzahl von konkaven Einheiten vorgesehen ist, wo ein Ausmaß der Änderung entlang der Entfernung zuzunehmen anstatt abzunehmen beginnt; und
eine Breite der Beinloch-Stretcheinheit mit den konkaven Einheiten in der Produktbreitenrichtung geringer ist als eine Breite der Beinloch-Stretcheinheit ohne konkave Einheiten in der Produktbreitenrichtung.

2. Wegwerfwindel nach Anspruch 1, wobei
die konkaven Einheiten neben der Mitte der Wegwerfwindel in der Produktlängsrichtung und in der rückwärtigen Tailleneinheit ausgebildet sind.

3. Wegwerfwindel nach Anspruch 2, wobei
zwischen den konkaven Einheiten eine konvexe Einheit, die in Richtung einer Außenseite in der Produktbreitenrichtung konvex ist, ausgebildet ist.

4. Wegwerfwindel nach einem der Ansprüche 1 bis 3, wobei
die Beinloch-Stretcheinheit entlang einem Endrand der Beinlocköffnung der Wegwerfwindel vorgesehen ist; und
der Seitenrand der Beinloch-Stretcheinheit in der Produktbreitenrichtung die wellige Form durch Bildung eines Wendepunktes in der Beinloch-Stretcheinheit ausbildet.

5. Wegwerfwindel nach einem der Ansprüche 1 bis 4, wobei
eine Breite der Beinloch-Stretcheinheit in der Produktbreitenrichtung in einem natürlichen Zustand auf 5 mm bis 45 mm eingestellt ist.

6. Wegwerfwindel nach einem der Ansprüche 1 bis 5, wobei
ein Verhältnis zwischen der Ausdehnung und der Schrumpfung der Beinloch-Stretcheinheit auf das 1,5-malige bis 2,4-malige eingestellt ist.

7. Wegwerfwindel nach einem der Ansprüche 1 bis 6, wobei
die Amplitude in der Produktbreitenrichtung bei einem Teil in einer in der Beinloch-Stretcheinheit ausgebildeten welligen Form auf 2,5 mm bis 20 mm eingestellt ist.

8. Wegwerfwindel nach einem der Ansprüche 1 bis 7, wobei
die an der rückwärtigen Tailleneinheit vorgesehene Seitenklappe ein Paar von Befestigungsbändern umfasst, die mit der vorderen Tailleneinheit zum Eingriff kommen können.

## Revendications

1. Couche jetable 10, comportant :
une unité avant au niveau de la taille 20 ;
une unité arrière au niveau de la taille 30 ;
une unité au niveau de l'entrejambe 25 ;
un corps absorbant verticalement long 15 comprenant un élément absorbant de retenue de liquide 40 ;
un rabat latéral 70 mis en oeuvre au niveau de chaque bord latéral dans une direction allant dans le sens de la largeur du produit du corps absorbant ;
une unité d'ouverture de trou pour jambe 35 qui est de forme concave vers un centre dans la direction allant dans le sens de la largeur du produit du corps absorbant, et qui est formée au niveau de chaque bord latéral dans la direction allant dans le sens de la largeur du produit du corps absorbant ; dans laquelle
la couche jetable comprend une unité extensible de trou pour jambe similaire à une feuille 100 mise en oeuvre au niveau d'une extrémité latérale, dans la direction allant dans le sens de la largeur du produit, du rabat latéral, s'étendant dans une direction allant dans le sens longitudinal du produit, et étant au moins partiellement extensible au moins dans la direction allant dans le sens longitudinal du produit ;
un bord latéral, dans la direction allant dans le sens de la largeur du produit, de l'unité extensible de trou pour jambe est en une forme ondulée dans la direction allant dans le sens longitudinal du produit ;
une distance entre le bord latéral, dans la direction allant dans le sens de la largeur du produit, de l'unité extensible de trou pour jambe et une ligne droite passant au travers d'un centre, dans la direction allant dans le sens de la largeur du produit, de la couche jetable et s'étendant de manière parallèle par rapport à la direction allant dans le sens longitudinal du produit, change de l'unité avant au niveau de la taille à l'unité arrière au niveau de la taille ;
une pluralité d'unités concaves où une quantité de changement le long de la distance commence à augmenter au lieu de diminuer sont mises en oeuvre de manière plus proche de l'unité arrière au niveau de la taille que par rapport à un centre, dans la direction allant dans le sens longitudinal du produit, de la couche jetable ; et
une largeur, dans la direction allant dans le sens de la largeur du produit, de l'unité extensible de trou pour jambe mise en oeuvre avec les unités concaves est inférieure par rapport à une largeur, dans la direction allant dans le sens de la largeur du produit, de l'unité extensible de trou pour jambe autre que les unités concaves.

2. Couche jetable selon la revendication 1, dans laquelle
les unités concaves sont formées de manière adjacente par rapport au centre, dans la direction allant dans le sens longitudinal du produit, de la couche jetable et sont formées dans l'unité arrière au niveau de la taille.

3. Couche jetable selon la revendication 2, dans laquelle
une unité convexe, qui est de forme convexe vers un côté extérieur dans la direction allant dans le sens de la largeur du produit, est formée entre les unités concaves.

4. Couche jetable selon l'une quelconque des revendications 1 à 3, dans laquelle
l'unité extensible de trou pour jambe est mise en oeuvre le long d'un bord d'extrémité de l'ouverture de trou pour jambe de la couche jetable ; et
le bord latéral, dans la direction allant dans le sens de la largeur du produit, de l'unité extensible de trou pour jambe forme la forme ondulée par la formation d'un point d'inflexion dans l'unité extensible de trou pour jambe.

5. Couche jetable selon l'une quelconque des revendications 1 à 4, dans laquelle
une largeur dans la direction allant dans le sens de la largeur du produit de l'unité extensible de trou pour jambe dans un état naturel est réglée de 5 mm à 45 mm.

6. Couche jetable selon l'une quelconque des revendications 1 à 5, dans laquelle
un rapport d'expansion et de contraction de l'unité extensible de trou pour jambe est réglé de 1,5 fois à 2,4 fois.

7. Couche jetable selon l'une quelconque des revendications 1 à 6, dans laquelle
une amplitude dans la direction allant dans le sens de la largeur du produit, au niveau d'une partie en une forme ondulée formée dans l'unité extensible de trou pour jambe, est réglée de 2,5 mm à 20 mm.

8. Couche jetable selon l'une quelconque des revendications 1 à 7, dans laquelle
le rabat latéral, mis en oeuvre au niveau de l'unité arrière au niveau de la taille, comprend une paire de rubans d'attache en mesure de se mettre en prise avec l'unité avant au niveau de la taille.
